(19) Europäisches Patentamt
European Patent Office
Office européen des brevets

(11) **EP 2 714 131 B1**

(12) **EUROPÄISCHE PATENTSCHRIFT**

(45) Veröffentlichungstag und Bekanntmachung des Hinweises auf die Patenterteilung:
**29.03.2017  Patentblatt 2017/13**

(21) Anmeldenummer: **12724908.4**

(22) Anmeldetag: **25.05.2012**

(51) Int Cl.:
***A61M 1/34*** (2006.01)

(86) Internationale Anmeldenummer:
**PCT/EP2012/002241**

(87) Internationale Veröffentlichungsnummer:
**WO 2012/159767 (29.11.2012 Gazette 2012/48)**

(54) **VERFAHREN ZUM BESTIMMEN EINES VOLUMENSTROMS IN EINER BLUTBEHANDLUNGSVORRICHTUNG, RECHENEIRICHTUNG UND BLUTBEHANDLUNGSVORRICHTUNG**

METHOD FOR DETERMINING A VOLUMETRIC FLOW IN A BLOOD TREATMENT APPARATUS, COMPUTING DEVICE AND BLOOD TREATMENT APPARATUS

PROCÉDÉ DE DÉTERMINATION D'UN DÉBIT VOLUMÉTRIQUE DANS UN DISPOSITIF DE TRAITEMENT DU SANG, DISPOSITIF DE CALCUL ET DISPOSITIF DE TRAITEMENT DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.05.2011  DE 102011103261
26.05.2011  US 201161490083 P
04.07.2011  DE 102011051549**

(43) Veröffentlichungstag der Anmeldung:
**09.04.2014  Patentblatt 2014/15**

(73) Patentinhaber: **Fresenius Medical Care Deutschland GmbH
61352 Bad Homburg (DE)**

(72) Erfinder: **GAGEL, Alfred
96123 Litzendorf (DE)**

(74) Vertreter: **Bobbert, Cornelius
Bobbert & Partner
Patentanwälte PartmbB
Postfach 1252
85422 Erding (DE)**

(56) Entgegenhaltungen:
**EP-A1- 1 681 068     WO-A1-00/23140
WO-A1-2008/125894     US-A- 5 660 722
US-A1- 2003 136 181**

**Beschreibung**

Die vorliegende Erfindung betrifft ein Verfahren zum Bestimmen eines Volumenstroms gemäß Anspruch 1. Sie betrifft ferner eine Blutbehandlungsvorrichtung gemäß Anspruch 13.

Bei manchen Verfahren der extrakorporalen Blutbehandlung wird dem verwendeten extrakorporalen Blutkreislauf und/oder einem Blutbehandlungsmodul, z. B. dem Blutfilter, eine Substituatflüssigkeit zugeführt. Es ist unter Praxisbedingungen nicht zuverlässig möglich, die Größe eines solchen Substituatflusses oder anderer Volumenströme zu ermitteln.

WO 2008/125894 A1 offenbart eine Blutbehandlungsvorrichtung mit einer mit einem Filter verbundenen Blutkammer. US 5 660 722 A offenbart eine Blutbehandlungsvorrichtung mit zwei seriengeschalteten Blutfiltern. US 2003/136181 A1 offenbart ein Verfahren zum Ermitteln eines Lecks im Kreislauf einer Blutbehandlungsvorrichtung.

Eine Aufgabe der vorliegenden Erfindung ist es, ein weiteres Verfahren zur Volumenstrombestimmung in einer Blutbehandlungsvorrichtung vorzuschlagen. Zudem soll eine entsprechende Blutbehandlungsvorrichtung angegeben werden.

Die erfindungsgemäße Aufgabe wird durch ein Verfahren mit den Merkmalen des Anspruchs 1 gelöst. Sie wird ferner durch eine Blutbehandlungsvorrichtung mit den Merkmalen des Anspruchs 13 gelöst.

Gemäß der vorliegenden Erfindung wird ein Verfahren zum Bestimmen wenigstens eines ersten Volumenstroms in einer Blutbehandlungsvorrichtung vorgeschlagen. Die Blutbehandlungsvorrichtung weist auf: einen Primärkreislauf zum Leiten des zu behandelnden Bluts, einen Sekundärkreislauf zum Leiten eines Fluids, das zur Blutbehandlung verwendet wird, und ein Blutbehandlungsmodul als Abschnitt des Primärkreislaufs und des Sekundärkreislaufs, das dazu vorgesehen ist, Fluide und/oder Stoffe zwischen dem Primärkreislauf und dem Sekundärkreislauf auszutauschen. Die Blutbehandlungsvorrichtung weist ferner eine Fluidverbindung zwischen dem Primärkreislauf und dem Sekundärkreislauf auf, vorgesehen zum Einbringen des ersten Volumenstroms aus dem Sekundärkreislauf in den Primärkreislauf. Die Fluidverbindung ist dabei als Schlauchleitung oder als Verbindungsleitung ausgebildet.

Das Verfahren umfasst zudem das Bestimmen des ersten Volumenstroms des Sekundärkreislaufs unter Einbeziehung eines ersten Druckmesswerts und eines zweiten Druckmesswerts im Sekundärkreislauf. Dabei ist erfindungsgemäß der erste Volumenstrom ein Teilvolumenstrom eines zweiten Volumenstroms. Erfindungsgemäß wird ferner der erste Volumenstrom als Funktion des Strömungswiderstands, des zweiten Volumenstroms und der Differenz aus zweitem Druckmesswert und erstem Druckmesswert bestimmt. Erfindungsgemäß ist der erste Volumenstrom ein Substituatstrom und der zweite Volumenstrom ein Dialysatfluss.

Die erfindungsgemäße Blutbehandlungsvorrichtung ist vorgesehen zum extrakorporalen Behandeln von Blut. Sie ist zudem zum Ausführen oder Durchführen des erfindungsgemäßen Verfahrens ausgestaltet und vorgesehen. Sie weist wenigstens auf oder ist hiermit verbunden: eine Recheneinrichtung, welche zum Ausführen des erfindungsgemäßen Verfahrens vorgesehen und/oder programmiert ist, einen Primärkreislauf zum Leiten des zu behandelnden Bluts, einen Sekundärkreislauf zum Leiten eines Fluids, das zur Blutbehandlung verwendet wird, ein Blutbehandlungsmodul als Abschnitt des Primärkreislaufs und des Sekundärkreislaufs, das dazu vorgesehen ist, Fluide und/oder Stoffe zwischen dem Primärkreislauf und dem Sekundärkreislauf auszutauschen, und eine Fluidverbindung zwischen dem Primärkreislauf und dem Sekundärkreislauf, vorgesehen zum Einbringen des ersten Volumenstroms aus dem Sekundärkreislauf in den Primärkreislauf.

Alle mit dem erfindungsgemäßen Verfahren erzielbaren Vorteile lassen sich in bestimmten erfindungsgemäßen Ausführungsformen ungeschmälert mit jedem der erfindungsgemäßen Gegenstände erzielen.

Ein digitales, insbesondere nicht-flüchtiges, Speichermedium, insbesondere in Form eines maschinenlesbaren Trägers, insbesondere in Form einer Diskette, CD, DVD oder EPROM, insbesondere mit elektronisch oder optisch auslesbaren Steuersignalen, kann derart mit einem programmierbaren Computersystem zusammenwirken, dass die maschinellen Schritte eines erfindungsgemäßen Verfahrens veranlasst werden.

Dabei können alle, einige oder manche der maschinell durchgeführten Schritte des erfindungsgemäßen Verfahrens veranlasst werden.

Ein Computerprogramm-Produkt kann einen auf einem maschinenlesbaren Träger gespeicherten Programmcode aufweisen zur Veranlassung der maschinellen Schritte des erfindungsgemäßen Verfahrens, wenn das Computerprogramm-Produkt auf einem Rechner abläuft. Unter einem Computerprogramm-Produkt kann beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Ein maschinenlesbarer Träger kann einen Träger bezeichnen, der mittels Software und/oder Hardware interpretierbare Daten oder Informationen enthält. Der Träger kann eine Diskette, eine CD, DVD, ein USB-Stick, eine Flashcard, eine SD-Karte und dergleichen sein.

Ein Computerprogramm kann einen Programmcode aufweisen zur Veranlassung der maschinellen Schritte eines erfindungsgemäßen Verfahrens, wenn das Computerprogramm auf einem Computer abläuft. Unter einem Computerprogramm kann beispielsweise ein physikalisches, vertriebsfähiges Software-Produkt verstanden werden, welches ein Programm aufweist.

Auch für das Computerprogramm-Produkt und das erfindungsgemäße Computerprogramm gilt, dass alle, einige oder manche der maschinell durchgeführten Schritte des erfindungsgemäßen Verfahrens veranlasst werden.

Erfindungsgemäße Ausführungsformen können eines oder mehrere der im Folgenden genannten Merkmale aufweisen. Erfindungsgemäße Ausführungsformen sind zudem Gegenstand der abhängigen Ansprüche.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll bestimmte erfindungsgemäße Ausführungsformen erläutern.

In manchen erfindungsgemäßen Ausführungsformen umfasst das erfindungsgemäße Verfahren das Bestimmen eines Volumenstroms des Sekundärkreislaufs stromauf des Blutbehandlungsmoduls unter Einbeziehung oder Berücksichtigung einer ersten Druckdifferenz, und/oder das Bestimmen eines Volumenstroms des Sekundärkreislaufs stromab des Blutbehandlungsmoduls unter Einbeziehung oder Berücksichtigung einer zweiten Druckdifferenz. Zudem wird erfindungsgemäß ein Volumenstrom stromauf und/oder ein Volumenstrom stromab des Blutbehandlungsmoduls jeweils mittels einer Druckdifferenzbestimmung an definierten Strömungswiderständen ermittelt.

In einigen erfindungsgemäßen Ausführungsformen ist der Primärkreislauf und/oder der Sekundärkreislauf ein geschlossener Kreislauf. In anderen erfindungsgemäßen Ausführungsformen ist der Primärkreislauf und/oder der Sekundärkreislauf ein offener Kreislauf oder ein nicht geschlossener Kreislauf.

In manchen erfindungsgemäßen Ausführungsformen ist der Sekundärkreislauf ein Dialysatkreislauf. In anderen erfindungsgemäßen Ausführungsformen ist er kein Dialysatkreislauf.

In bestimmten erfindungsgemäßen Ausführungsformen ist ein Volumenstrom eine Flussrate.

In manchen erfindungsgemäßen Ausführungsformen ist das Bestimmen ein quantitatives Bestimmen. In anderen erfindungsgemäßen Ausführungsformen ist das Bestimmen ein qualitatives Bestimmen. Letzteres kann z. B. das Angeben des ersten Volumenstroms als Anteil eines Gesamtvolumenstroms umfassen.

In einigen erfindungsgemäßen Ausführungsformen werden oder sind der erste Druckmesswert und der zweite Druckmesswert an verschiedenen Messorten innerhalb des Sekundärkreislaufs gemessen.

In manchen erfindungsgemäßen Ausführungsformen betreffen die erste Druckdifferenz und die zweite Druckdifferenz Druckdifferenzen innerhalb des Sekundärkreislaufs.

In manchen erfindungsgemäßen Ausführungsformen befindet sich der erste Messort stromauf eines jeden Blutbehandlungsmoduls, z. B. stromauf eines jeden Dialysefilters, und der zweite Messort befindet sich stromab eines jeden Blutbehandlungsmoduls, z. B. stromab eines jeden Dialysefilters. Umfasst der Dialysefilterabschnitt mehrere, getrennt vorliegende Dialysefilter, so können sie in manchen erfindungsgemäßen Ausführungsformen als gemeinsamer Dialysefilter betrachtet werden.

In einigen erfindungsgemäßen Ausführungsformen dienen die Drucksensoren nicht zur Bestimmung des jeweiligen Filtratflusses oder eines anderen Flusses durch die Membran eines Blutbehandlungsfilters hindurch.

In bestimmten erfindungsgemäßen Ausführungsformen gibt der erste Druckmesswert einen Druck im Sekundärkreislauf an einer ersten Druckmessstelle stromauf des Blutbehandlungsmoduls an, und zwar stromab einer Aufteilung, Trennung oder Abzweigung des ersten Volumenstroms aus einem zweiten - vorzugsweise größeren - Volumenstrom.

In manchen erfindungsgemäßen Ausführungsformen wird der Volumenstrom in einem Dialysierflüssigkeitsfilter oder einem Online-Filter aufgeteilt. Ein Online-Filter kann ein Filter sein, in welchem die Flüssigkeitsmenge, die das Dialysegerät zur Substitution benötigt oder vorsieht, und welche dem geschlossenen System vor dem Dialysator entnommen wird, erneut gefiltert wird.

In einigen erfindungsgemäßen Ausführungsformen gibt der zweite Druckmesswert einen Druck im Sekundärkreislauf an einer zweiten Druckmessstelle an, welche stromab des Blutbehandlungsmoduls liegt.

In bestimmten erfindungsgemäßen Ausführungsformen wird der erste Volumenstrom mittels einer Pumpe, z. B. mittels einer Substituatpumpe, dem zweiten Volumenstrom entnommen und in den Primärkreislauf oder in das Blutbehandlungsmodul gefördert.

In einigen erfindungsgemäßen Ausführungsformen des Verfahrens wird ein Strömungswiderstand zwischen der ersten Druckmessstelle und der zweiten Druckmessstelle als Funktion des zweiten Volumenstroms und der Differenz aus zweitem Druckmesswert und erstem Druckmesswert bestimmt.

In manchen erfindungsgemäßen Ausführungsformen wird die Funktion des Strömungswiderstands im Bereich wenigstens eines Arbeitspunktes linearisiert.

Ein Arbeitspunkt kann durch einen Volumenstrom und eine Druckdifferenz angegeben werden. Eine Linearisierung liegt beispielsweise dann vor, wenn benachbarte Arbeitspunkte in einem vorgegebenen Volumenstrom-/Druckdifferenzbereich auf einer Geraden liegen, die mathematisch beschrieben werden kann. Die Korrelation der Messpunkte mit der Geraden kann durch den Korrelationskoeffizienten $R^2$ angegeben werden. Eine hinreichend genaue Linearisierung kann z. B. durch einen Korrelationskoeffizienten R$^2$ größer oder gleich 0,995 vorgegeben werden.

In manchen erfindungsgemäßen Ausführungsformen ist der erste Volumenstrom klein gegenüber dem zweiten Volumenstrom. Der erste Volumenstrom ist in einigen erfindungsgemäßen Ausführungsformen dann als klein zu betrachten, wenn er max. 4 % oder max. 8 % des zweiten Volumenstroms beträgt.

In manchen erfindungsgemäßen Ausführungsformen wird der funktionelle Zusammenhang eines Druckverlusts zwischen erster Druckmessstelle und einem Abschnitt innerhalb des Blutbehandlungsmoduls in Abhängigkeit des zweiten Volumenstroms im Sekundärkreislauf werksseitig vor Beginn der Blutbehandlung bestimmt oder wurde auf diese Weise bestimmt.

In einigen erfindungsgemäßen Ausführungsformen ist der Abschnitt innerhalb des Blutbehandlungsmoduls dessen Mitte.

Die Mitte des Blutbehandlungsmoduls kann auf dessen durchströmte Strecke auf der Seite des Primärkreislaufs oder Sekundärkreislaufs bezogen sein.

Der erste Volumenstrom kann hierbei auf Null gesetzt sein oder werden.

In manchen erfindungsgemäßen Ausführungsformen wird der funktionelle Zusammenhang eines Druckverlusts zwischen erster Druckmessstelle und einem Abschnitt innerhalb des Blutbehandlungsmoduls in Abhängigkeit des zweiten Volumenstroms im Sekundärkreislauf als Funktion eines Druckverlusts bestimmt oder wurde auf diese Weise bestimmt. Dieser Druckverlust entsteht zwischen erster Druckmessstelle und zweiter Druckmessstelle und hängt vom zweiten Volumenstrom ab. In diesem Fall wird der Druckverlust infolge des Strömungswiderstands ab der ersten Druckmessstelle bis zu einem Abschnitt innerhalb des Blutbehandlungsmoduls extrapoliert, um auch jenen Strömungswiderstand zu umfassen, welcher bis zur zweiten Druckmessstelle entsteht. Dies ist in der Regel vor allem dann zulässig, wenn beide Strömungsabschnitte gleich oder ähnlich aufgebaut sind und daher gleiche oder ähnliche Strömungswiderstände aufweisen.

In einigen erfindungsgemäßen Ausführungsformen wird - oder wurde zu einem Zeitpunkt vor Verfahrensbeginn - der Druckverlust zwischen erster Druckmessstelle und zweiter Druckmessstelle - ausschließlich oder nicht ausschließlich - unter Berücksichtigung des Druckverlusts zwischen erster Druckmessstelle und zweiter Druckmessstelle in Abhängigkeit des zweiten Volumenstroms und des Volumenstroms, der als UF-Rate oder Transmembranfluss im Behandlungsmodul vom Primärkreislauf in den Sekundärkreislauf übertritt, bestimmt.

Die Summe aus dem zweiten Volumenstrom und dem Volumenstrom, der im Behandlungsmodul vom Primärkreislauf in den Sekundärkreislauf transportiert wird, entspricht, unter der Voraussetzung, dass der erste Volumenstrom deutlich kleiner ist als der zweite Volumenstrom, z. B. max. 10 %, im Wesentlichen dem Volumenstrom, der im zweiten Abschnitt, also vom Blutbehandlungsmodul bis zur zweiten Druckmessstelle, fließt. Da dieser Volumenstrom jedoch für die Bestimmung des Strömungswiderstands und damit des Druckverlusts für die Gesamtstrecke, also von erster bis zweiter Druckmessstelle, unrealistisch hoch ist, wird ein Druckverlustanteil des ersten Abschnitts abgezogen. Dieser Anteil kann näherungsweise durch das totale Differential bestimmt werden:

$$\left(\frac{dF_{1,m}}{dQ}\right)_{(Q_{BK} + Q_{UF} + Q_d)/2} * Q_{sub}$$

mit:

$F_{l,m}$ - Druckverlust als Funktion des Volumenstroms zwischen erster Druckmessstelle und der Mitte des Blutbehandlungsmoduls;

$Q_{BK}$ - zweiter Volumenstrom;

$Q_{UF}$ - Volumenstrom, der im Blutbehandlungsmodul aus dem Primärkreislauf in den Sekundärkreislauf übertritt; und

$Q_d$ - zweiter Volumenstrom abzüglich des ersten Volumenstroms.

In manchen erfindungsgemäßen Ausführungsformen wird ein Leck oder eine Leckrate, welche sich aus unterschiedlich großen Volumenströmen im Sekundärkreislauf stromab und stromauf des Blutbehandlungsmoduls ergibt oder hiermit einhergeht, bestimmt. Zu dieser Bestimmung wird ein funktioneller Zusammenhang der folgenden Parameter ermittelt oder angegeben, zu welchen zählen: der erste Druckmesswert; der zweite Druckmesswert; der Strömungswiderstand zwischen erster Druckmessstelle und einem Abschnitt innerhalb des Blutbehandlungsmoduls; der Strömungswiderstand zwischen dem Abschnitt innerhalb des Blutbehandlungsmoduls und der zweiten Druckmessstelle; der erste Volumenstrom; der zweite Volumenstrom; und - optional - ein dritter Volumenstrom. Der dritte Volumenstrom entspricht einem Fluidaustausch oder Fluidübertritt über eine Membran zwischen Primärkreislauf und Sekundärkreislauf des Blutbehandlungsmoduls.

Unter einer funktionellen Beziehung wird in bestimmten erfindungsgemäßen Ausführungsformen eine Funktion, ein Zusammenhang, ein mathematischer Zusammenhang, eine Formel oder dergleichen verstanden.

Die Dichtigkeit des Sekundärkreislaufs gegenüber einem Äußeren des Sekundärkreislaufs zwischen einem ersten Ventil stromauf des Blutbehandlungsmoduls und einem zweiten Ventil stromab des Blutbehandlungsmoduls kann ermittelt werden. Dies umfasst das Schließen des ersten Ventils und das Schließen des zweiten Ventils. Anschließend wird das Fluidsystem stromauf des ersten Ventils und/oder stromab des zweiten Ventils mit einem Überdruck beaufschlagt. Dies kann beispielsweise mittels der Dialysatpumpe erfolgen. Dabei wird überprüft oder überwacht, ob sich der Überdruck im Fluidsystem innerhalb einer vorgegebenen Zeitspanne verändert. Ergänzend oder alternativ wird überprüft oder überwacht, ob sich der Überdruck verändert oder abbaut. Wird ein Druckabfall oder ein Druckabbau jenseits eines vorgegebenen Druckbereichs erkannt, so wird der Sekundärkreislauf oder sein auf Dichtigkeit überprüfter Abschnitt als nicht dicht erkannt. Die Überprüfung auf Druckabfall kann mittels der ersten Druckmessstelle und/oder mittels der zweiten Druckmessstelle erfolgen.

Optional kann bei diesen Ausführungsformen geprüft oder sichergestellt werden, ob der erste Volumenstrom Null ist. Ein Sicherstellen, dass der erste Volumenstrom Null beträgt, erfolgt beispielsweise dadurch, dass mittels der Substituatpumpe kein Fluss erzeugt wird.

In manchen erfindungsgemäßen Ausführungsformen wird in dem Fall, dass der festgestellte Druckabfall den vorgegebenen Druckbereich verlässt, ein Leck im Sekundärkreislauf festgestellt und die Blutbehandlungsvorrichtung oder auch nur eine Ultrafiltration aus Gründen der Patientensicherheit gestoppt.

In manchen erfindungsgemäßen Ausführungsformen wird bei unterschiedlich großen Volumenströmen des Sekundärkreislaufs stromab und stromauf des Blutbehandlungsmoduls für den Fall, dass der Druckabfall den vorgegebenen Druckbereich nicht verlässt, ein Offset-Fehler der Volumenstrombestimmung festgestellt. Dieser Offset kann in weitere Messungen einfließen und/oder bei der Behandlung des Patienten berücksichtigt werden, ohne dass die Blutbehandlung abgebrochen werden muss.

Definierte oder bekannte Strömungswiderstände können an im Kreislauf vorhandenen Ventilen, Blenden oder anderen Komponenten auftreten.

In manchen erfindungsgemäßen Ausführungsformen wird ein Offset für Volumenstrombestimmungen ermittelt. Dies umfasst das Überprüfen, insbesondere nach einer oben beschriebenen Vorgehensweise, ob der Sekundärkreislaufs oder ein betrachteter Abschnitt hiervon dicht gegenüber einer äußeren Umgebung und/oder einem weiteren Leitungsabschnitt ist. Es umfasst ferner das Überprüfen oder das Sicherstellen, ob bzw. dass kein Fluid zwischen Primärkreislauf und Sekundärkreislauf, beispielsweise über das Blutbehandlungsmodul, ausgetauscht wird. Ferner umfasst dieses Vorgehen das Ermitteln einer Differenz zwischen dem Volumenstrom stromauf und dem Volumenstrom stromab des Blutbehandlungsmoduls im Sekundärkreislauf. Diese kann beispielsweise nach einem oben beschriebenen Verfahren ermittelt werden.

Schließlich umfasst dieses Vorgehen das Festlegen der Differenz zwischen den Volumenströmen - oder eines hiermit korrelierenden Wertes - als Offset.

In bestimmten erfindungsgemäßen Ausführungsformen umfasst das Verfahren das Ermitteln eines Fluidaustauschs oder Fluidübertritts - oder eines Wertes hierfür - im Blutbehandlungsmodul zwischen Primärkreislauf und Sekundärkreislauf. Ein solcher Fluidaustausch gilt als ermittelt, wenn unterschiedlich große Volumenströme im Sekundärkreislauf stromab und stromauf des Blutbehandlungsmoduls festgestellt werden.

Ein solcher Fluidaustausch kann dem gesamten Transmembranfluss, welcher im Wesentlichen der Ultrafiltrationsrate (UF) entspricht, gleichzusetzen sein.

Wird ein solcher Fluidaustausch festgestellt, so kann in einem folgenden Schritt geprüft werden, ob ein Offset oder eine Leckage vorliegt. Die Ergebnisse dieser Überprüfung fließen in die weitere Dialysebehandlung ein, indem entweder ein Offset eingestellt oder die Behandlung des Patienten abgebrochen wird.

In einigen erfindungsgemäßen Ausführungsformen ist das Bestimmen des ersten, zweiten, dritten und/oder vierten Druckmesswerts Teil des Verfahrens, in anderen nicht. Das Bestimmen der Druckwerte kann direkt, z. B. durch direktes Messen, oder indirekt erfolgen. Von einem Druckmesswert ist erfindungsgemäß daher selbst dann die Rede, wenn der Druck nicht gemessen, sondern anderweitig, z. B. durch Errechnen aus anderen Größen, ermittelt oder festgelegt wurde.

Die Recheneinrichtung der Blutbehandlungsvorrichtung kann in bestimmten erfindungsgemäßen Ausführungsformen eine oder mehrere Steuer- oder Regeleinheiten zum Steuern bzw. Regeln einer Blutbehandlungsvorrichtung aufweisen oder hiermit verbunden sein.

In einigen erfindungsgemäßen Ausführungsformen kann die Recheneinrichtung der Blutbehandlungsvorrichtung geeignet und konfiguriert zum qualitativen Bestimmen des ersten Volumenstroms des Sekundärkreislaufs als Anteil eines zweiten Volumenstroms sein.

In manchen erfindungsgemäßen Ausführungsformen weist die Blutbehandlungsvorrichtung die zum Durchführen des erfindungsgemäßen Verfahrens erforderlichen und hierfür vorgesehenen oder konfigurierten Einrichtungen auf. Dies gilt insbesondere für die im Zusammenhang mit dem hierin offenbarten Verfahren genannten Einrichtungen. Dabei kann die Blutbehandlungsvorrichtung - wie jede andere Vorrichtung gemäß der vorliegenden Erfindung - wenigstens eine oder mehrere geeignete Einrichtung(en) aufweisen, welche geeignet und/oder konfiguriert und/oder ausgestaltet sind, so dass einer, mehrere oder alle der hierin genannten Verfahrensschritte mittels der betreffenden Vorrichtung durchgeführt werden können.

In einigen erfindungsgemäßen Ausführungsformen ist die Fluidverbindung keine Filtermembran und/oder weist keine Filtermembran auf.

In manchen erfindungsgemäßen Ausführungsformen weist die Blutbehandlungsvorrichtung wenigstens oder genau zwei im Sekundärkreislauf liegende Druckmessstellen stromauf des Blutbehandlungsmoduls und/oder nach Abgang der Fluidverbindung aus dem Sekundärkreislauf auf.

Ergänzend oder alternativ weist die Blutbehandlungsvorrichtung in diesen Ausführungsformen zwei im Sekundärkreislauf gelegene Druckmessstellen stromab des Blutbehandlungsmoduls auf.

In manchen der zuletzt genannten erfindungsgemäßen Ausführungsformen liegen die jeweils wenigstens zwei Druckmessstellen, z. B. die erste und die dritte, oder die zweite und die vierte, stromauf bzw. stromab eines bekannten Strömungswiderstands. So können die erste und die dritte Druckmessstelle stromauf bzw. stromab des ersten Ventils liegen oder messen. Ferner können die zweite und die vierte Druckmessstelle stromauf bzw. stromab des zweiten Ventils liegen oder messen.

In bestimmten erfindungsgemäßen Ausführungsformen ist die Blutbehandlungsvorrichtung eine Hämofiltrationsvorrichtung oder eine Hämodiafiltrationsvorrichtung.

In manchen erfindungsgemäßen Ausführungsformen ist das Blutbehandlungsmodul ein Blutfilter, ein Dialysefilter oder ein Hämodialysefilter.

Manche, einige oder bestimmte erfindungsgemäße Ausführungsformen weisen einen, manche oder alle der folgenden Vorteile auf:

Erfindungsgemäß bedarf es vorteilhafterweise keiner Vorrichtung, um den Substituatfluss oder den Dialysatorfluss direkt zu messen.

Die Hämodialyse ist ein Verfahren zur Entfernung von harnpflichtigen Substanzen und zum Flüssigkeitsentzug aus dem Blut von an Niereninsuffizienz leidenden Patienten. Bei der Hämodialyse wird das Blut des Patienten außerhalb des Körpers in einem Dialysator gereinigt. Der Dialysator weist eine Blutkammer und eine Dialysierflüssigkeitskammer auf, die mittels einer semipermeablen Membran getrennt sind. Während der Behandlung strömt Blut des Patienten durch die Blutkammer. Um das Blut effektiv von harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleineren molekularen Substanzen durch die Membran des Dialysators im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe, durch einen hohen Flüssigkeitsstrom (Konvektion) durch die Membran des Dialysators effektiv entfernt. Bei der Hämofiltration dient der Dialysator als Filter. Eine Kombination aus beiden Verfahren ist die Hämodiafiltration (HDF).

Bei der Hämo(dia)filtration wird ein Teil des durch die Membran des Dialysators abgezogenen Serums durch eine sterile Substitutionsflüssigkeit ersetzt, die im Allgemeinen entweder stromauf des Dialysators oder stromab des Dialysators dem extrakorporalen Blutkreislauf zugeführt wird. Die Zufuhr der Substitutionsflüssigkeit stromauf des Dialysators wird auch als Prädilution bezeichnet, die Zufuhr stromab des Dialysators als Postdilution.

Zwar kann der Substituatfluss auch indirekt ermittelt werden. Dies könnte in Abhängigkeit des Typs der verwendeten Substituatpumpe beispielsweise indirekt aus der Drehzahl und dem eingangsdruckabhängigen Schlagvolumen der Substituatpumpe, die als okkludierende Schlauchrollenpumpe ausgeführt sein kann, berechnet werden. Der reale Substituatfluss kann aufgrund von z. B. Schlauch-Kinking (Abknicken des Schlauchs) oder unvollständiger Okklusion des Pumpschlauchs und dergleichen allerdings mehr oder weniger stark vom berechneten Substituatfluss abweichen. Derartige Fehler oder Abweichungen zwischen indirekt ermitteltem und tatsächlichem Substituatfluss sind regelmäßig durch bekannte Überwachungsmethoden wie beispielsweise der TMP-Überwachung nicht festzustellen: Bei der TMP-Überwachung (TMP - transmembrane pressure) ist die Bilanziergenauigkeit bei derartigen Abweichungen nicht beeinträchtigt. Hier schafft die vorliegende Erfindung in einigen Ausführungsformen vorteilhaft Abhilfe.

Bei HDF-Behandlungen wird die Clearance bei Mittelmolekülen vom Substituatfluss bestimmt. Abweichungen im Substituatfluss schlagen sich somit in der verabreichten Dialysedosis der Behandlung nieder. Die Genauigkeit und die Sicherheit der Behandlung des Patienten lassen sich bei Kenntnis des genauen Substituatflusses somit in manchen Ausführungsformen vorteilhaft steigern.

Ein Vorteil einiger erfindungsgemäßer Ausführungsformen liegt in der Genauigkeit, mit welcher der Substituatfluss bestimmt werden kann. Die erzielbare Genauigkeit beruht u. a. darauf, dass die Strömungswiderstände $R_1$ und $R_2$ durch nicht auszutauschende Hydraulik-Komponenten der zur Behandlung verwendeten Blutbehandlungsvorrichtung bedingt sind, welche zur erfindungsgemäßen Berechnung des Substituatflusses verwendet werden. Damit beruht die Berechnung nicht auf dem Strömungswiderstand von zur individuellen Behandlungssitzung eigens ausgesuchten und verwendeten Disposables (z. B. Dialysatoren mit teils sehr unterschiedlichen Ultrafiltrationskoeffizienten), deren Strömungsunterschiede sich von Typ zu Typ und sogar von Disposable zu Disposable desselben Typs unterscheiden können. Die erzielbare Genauigkeit beruht u. a. auch darauf, dass alle bei der Volumenstrombestimmung berücksichtigten Fluide solche sind, deren Eigenschaften bekannt und zudem unveränderlich sind. Dies trifft beispielsweise auf Dialysat zu, auf welches sich die hierin beschriebenen Berechnungen stützen. Dieselbe Annahme trifft hingegen nicht auch auf andere Fluide wie etwa Blut zu. Dessen Viskosität kann sich aufgrund geänderter Zusammensetzung (Hkt, TP, Fibrinogen, ...) von Behandlung zu Behandlung ändern. Zudem kann sich die Viskosität von Blut auch innerhalb einer Behandlung aufgrund des Wasserentzugs ändern. Bei Hämodiafiltrationsbehandlungen verändert sich zudem der effektive UF-Leistung des Dialysators durch den Aufbau einer reversiblen Sekundärmembran in Abhängigkeit der Substituatrate und der Behandlungsart (Prä-/Post-Dilution). Auch dies spielt beim erfindungsgemäßen Verfahren vorteilhaft keine Rolle.

Während der Dialysebehandlung können mittels der Druckdifferenzmessung und anhand der zuvor ermittelten Strömungswiderstandsmessung kontinuierlich der Transmembranfluss oder die UF-Rate ermittelt werden. Auch dies kann vorteilhaft zur Erhöhung der Patientensicherheit beitragen.

Im Folgenden wird die vorliegende Erfindung unter Bezugnahme auf die beigefügten Figuren rein exemplarisch beschrieben. In den Figuren bezeichnen identische Bezugszeichen gleiche oder identische Elemente. Mittels der Pfeile wird stets eine Strömungsrichtung des betrachteten Fluids angegeben. Es gilt:

Fig. 1    zeigt einen Ausschnitt einer schematisch vereinfachten Blutbehandlungsvorrichtung mit Primärkreislauf und Sekundärkreislauf gemäß einer ersten Ausführungsform der vorliegenden Erfindung;

Fig. 2    zeigt eine Messkurve einer Druckdifferenz im Sekundärkreislauf zwischen einer ersten und einer zweiten Druckmessstelle über einem zweiten Volumenstrom zur Bestimmung eines Strömungswiderstands;

Fig. 3    zeigt eine Messkurve des doppelten zweiten Volumenstroms abzüglich eines ersten Volumenstroms über der Druckdifferenz zwischen der ersten und der zweiten Druckmessstelle im Sekundärkreislauf; und

Fig. 4    zeigt schematisch den Teilausschnitt des Sekundärkreislaufs aus Fig. 1 der Blutbehandlungsvorrichtung gemäß einer ersten Ausführungsform der vorliegenden Erfindung mit zwei weiteren Druckmessstellen.

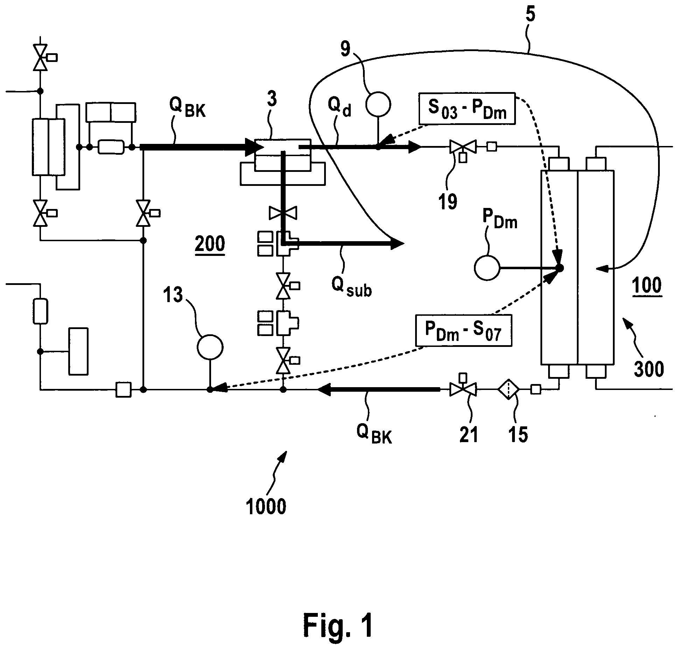

**Fig. 1** zeigt schematisch einen Ausschnitt einer Hämodiafiltrationsvorrichtung (im Folgenden kurz HDF genannt) als Beispiel einer erfindungsgemäßen Blutbehandlungsvorrichtung 1000 mit einem Primärkreislauf 100 und einem Sekundärkreislauf 200. Primärkreislauf 100 und Sekundärkreislauf 200 durchströmen jeweils ein gemeinsames Blutbehandlungsmodul 300, hier beispielhaft als Blutfilter oder Dialysator dargestellt.

Ein erster Volumenstrom, im Folgenden als Substituatfluss $Q_{sub}$ bezeichnet, und ein dritter Volumenstrom, im Folgenden als Dialysatorfluss $Q_d$ bezeichnet, entstehen durch Aufteilung eines zweiten Volumenstroms, im Folgenden als Dialysatfluss $Q_{BK}$ bezeichnet. Die Aufteilung erfolgt in einem Filter 3 des Sekundärkreislaufs 200.

Der Substituatfluss $Q_{sub}$ wird dem Dialysatfluss $Q_{BK}$ beispielsweise mittels einer Substituatpumpe entnommen. Der Substituatfluss $Q_{sub}$ wird mittels einer Fluidleitung oder Fluidverbindung 5, welche in Fig. 1 mittels Pfeil nur angedeutet ist, in den Primärkreislauf 100, einen extrakorporalen Blutkreislauf, eingebracht.

Der Dialysatorfluss $Q_d$ durchströmt das Blutbehandlungsmodul 300 im Sekundärkreislauf 200.

Diese Volumenverteilungen und -beziehungen lassen sich mittels folgender Gleichung darstellen:

$$Q_{BK} = Q_d + Q_{sub} \tag{1}$$

$Q_{BK}$ - Dialysatfluss
$Q_d$ - Dialysatorfluss
$Q_{sub}$ - Substituatfluss

Bevor der Dialysatfluss $Q_{BK}$ wie oben beschrieben aufgeteilt wird, durchströmt er im vorliegenden Beispiel eine Bilanzierkammer des Sekundärkreislaufs 200. Die Bilanzierkammer erlaubt die Bestimmung des Dialysatflusses $Q_{BK}$ als Funktion eines kalibrierten Bilanzierkammervolumens und einer Ladezeit der Bilanzierkammer:

$$Q_{BK} = \frac{V_{BK}}{T_{BK}}$$

mit:

$Q_{BK}$ - Dialysatfluss;
$V_{BK}$ - kalibriertes Bilanzierkammervolumen; und
$T_{BK}$ - Ladezeit der Bilanzierkammer.

Im vorliegenden Ausführungsbeispiel wird eine fehlerfrei arbeitende Vorrichtung betrachtet, bei der keine fehlerbehaftete Fehlbilanzierung oder Bilanzierung auftritt. Zudem wird angenommen, dass die Ultrafiltrationsrate $Q_{UF}$ ("UF-Rate") gemessen am Substituatfluss $Q_{sub}$ vernachlässigbar klein ist.

Stromab des Filters 3 wird der erste Druckmesswert $S_{03}$ des Dialysatorflusses $Q_d$ mittels eines ersten Drucksensors 9 gemessen. Der Druckwert in der Mitte des Blutbehandlungsmoduls 300 wird als $P_{Dm}$ bezeichnet. Somit entspricht die Druckdifferenz des Dialysatorflusses $Q_d$ zwischen der ersten Druckmessstelle 9 und der Mitte des Blutbehandlungsmoduls 300 dem Ausdruck $S_{03}$ - $P_{Dm}$.

Zwischen erster Druckmessstelle 9 und der Mitte des Dialysators 300 besteht ein Strömungswiderstand $R_1$. Der Strömungswiderstand $R_1$ ist eine Funktion des Dialysatorflusses $Q_d$:

$$R_1 = f\left(Q_d\right)$$

mit:

$R_1$ - Strömungswiderstand zwischen erster Druckmessstelle 9 und der Mitte des Dialysators 300; und
$Q_d$ - Dialysatorfluss.

Somit ergibt sich für die Druckdifferenz $S_{03}$ - $P_{Dm}$:

$$S_{03} - P_{Dm} = Q_d * R_1 = \left(Q_{BK} - Q_{sub}\right) * R_1 \qquad (2)$$

In diesem Ausführungsbeispiel wird der Substituatfluss $Q_{sub}$, der in den Dialysator 300 hineinströmt, vollständig aus dem Primärkreislauf 100 erneut in den Sekundärkreislauf 200 übertragen bzw. durch die im Dialysator 300 befindliche Membran transportiert. Daher tritt der Dialysatfluss $Q_{BK}$ am Ausgang des Blutbehandlungsmoduls 300 wieder auf Seiten des Sekundärkreislaufs 200 aus.

Weiter stromab des Dialysators 300 wird im Sekundärkreislauf 200 an einem zweiten Drucksensor 13 ein zweiter Druckmesswert $S_{07}$ gemessen. Somit ergibt sich die Druckdifferenz zwischen der Mitte des Blutbehandlungsmoduls 300 und dem zweiten Drucksensor 13 zu $P_{Dm}$ - $S_{07}$.

Analog zu $R_1$ wird ferner ein Strömungswiderstand $R_2$ eingeführt, er gibt den Strömungswiderstand an, der zwischen der Mitte des Dialysators 300 und der zweiten Druckmessstelle 13 vorliegt. Es gilt:

$$P_{Dm} - S_{07} = Q_{BK} * R_2 \qquad (3)$$

Addition der Gleichungen (2) und (3) ergibt:

$$S_{03} - S_{07} = Q_d * R_1 + Q_{BK} * R_2 = \left(Q_{BK} - Q_{sub}\right) * R_1 + Q_{BK} * R_2 \qquad (4a)$$

bzw.

$$S_{03} - S_{07} = Q_{BK} * \left(R_1 + R_2\right) - Q_{sub} * R_1 \qquad (4b)$$

Hieraus lässt sich der Substituatfluss $Q_{sub}$ berechnen:

$$Q_{sub} = Q_{BK} * \left(1 + \frac{R_2}{R_1}\right) - \frac{S_{03} - S_{07}}{R_1} \qquad (5)$$

Die Strömungswiderstände $R_1$ und $R_2$ sind ausschließlich durch Komponenten des Sekundärkreislauf 200 bedingt. Sie alle tragen in der Praxis in etwa gleich zum Strömungswiderstand bei, mit Ausnahme eines Dialysatfilters 15, der allerdings nur einen geringen Druckabfall erzeugt. Daher werden die Strömungswiderstände $R_1$ und $R_2$ in erster Näherung als gleich groß angenommen. Mit der Vereinfachung $R_1 = R_2 = R$ ergibt sich:

$$S_{03} - S_{07} = R * \left(2 * Q_{BK} - Q_{sub}\right) \qquad (6)$$

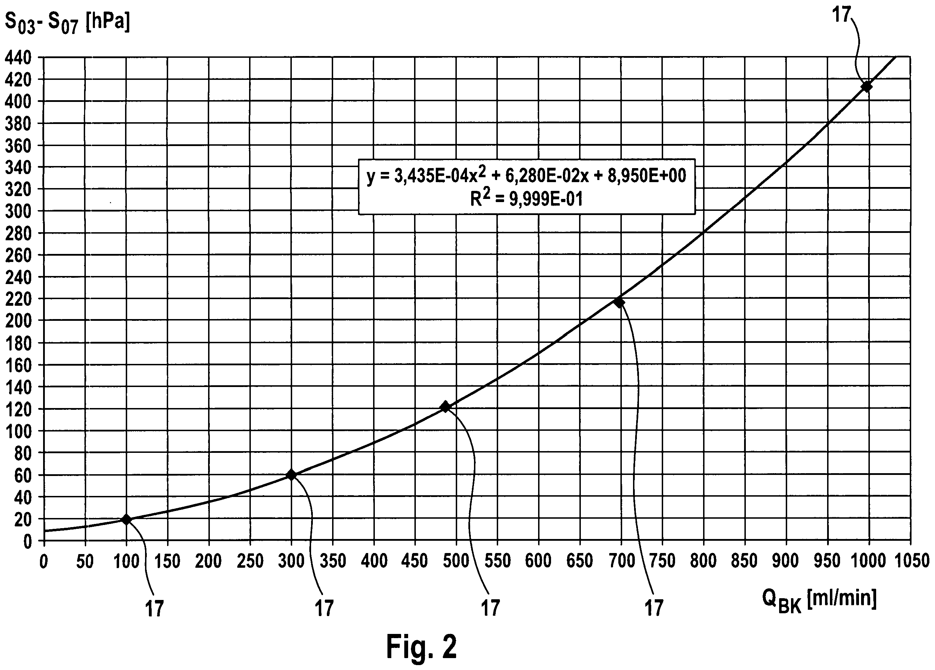

**Fig. 2** zeigt eine Messkurve der Druckdifferenz zwischen dem an der ersten Druckmessstelle 9 gemessenen ersten Druckmesswert $S_{03}$ und dem an der zweiten Druckmessstelle 13 gemessenen zweiten Druckmesswert $S_{07}$ über dem Dialysatfluss $Q_{BK}$ im Sekundärkreislauf 200. Der Substituatfluss $Q_{sub}$ beträgt Null ($Q_{sub}$ = 0 ml/min). Der Blutfluss im Primärkreislauf beträgt $Q_b$ = 450 ml/min.

In Fig. 2 sind fünf Messpunkte 17 eingezeichnet. Die Messpunkte wurden zwischen den Druckdifferenzwerten 20 hPa bis 420 hPa und bei Dialysatflüssen $Q_{BK}$ zwischen 100 ml/min und 1.000 ml/min aufgenommen. Durch diese Messpunkte wurde ein Polynom 2. Grades mit der angegebenen Funktion y = f(x$^2$) als Kalibrierfunktion gelegt. Der Korrelationskoeffizient beträgt $R^2$ = 0,9999.

Die Darstellung der Fig. 2 lässt sich angeben als:

$$S_{03} - S_{07} = F_{1,2}\left(Q_{BK}\right)$$

mit:

$F_{1,2}(Q_{BK})$ - Funktion des Dialysatflusses $Q_{BK}$ zwischen der ersten Druckmessstelle 9 und der zweiten Druckmessstelle 13

Nach Gleichung (6) kann der Strömungswiderstand R jederzeit, d. h. z. B. während einer Vorbereitung oder einem Behandlungseinsatz der Blutbehandlungsvorrichtung 1000, online in der Blutbehandlungsvorrichtung 1000 gemessen werden, indem die Substituatpumpe ausgeschaltet ($Q_{sub}$ = 0) oder konstant gehalten und der Dialysatfluss $Q_{BK}$ variiert wird:

$$R = \frac{S_{03} - S_{07}}{2 * Q_{BK}} \qquad (7)$$

Bedingt durch ein erstes Ventil 19, welches stromauf des Blutbehandlungsmoduls 300 im Sekundärkreislauf 200 angeordnet ist, und durch ein zweites Ventil 21, welches stromab des Blutbehandlungsmoduls 300 im Sekundärkreislauf 200 angeordnet ist, ergibt sich ein nichtlinearer Verlauf für den Strömungswiderstand R. Da der Substitutionsfluss $Q_{sub}$ üblicherweise klein gegenüber dem Dialysatfluss $Q_{BK}$ ist, kann der Strömungswiderstand R folgendermaßen näherungsweise durch Variation von $Q_{BK}$ um den Arbeitspunkt bestimmt werden:

$$R \cong \frac{\Delta\left(S_{03} - S_{07}\right)}{2 * \Delta Q_{BK}} \qquad (8)$$

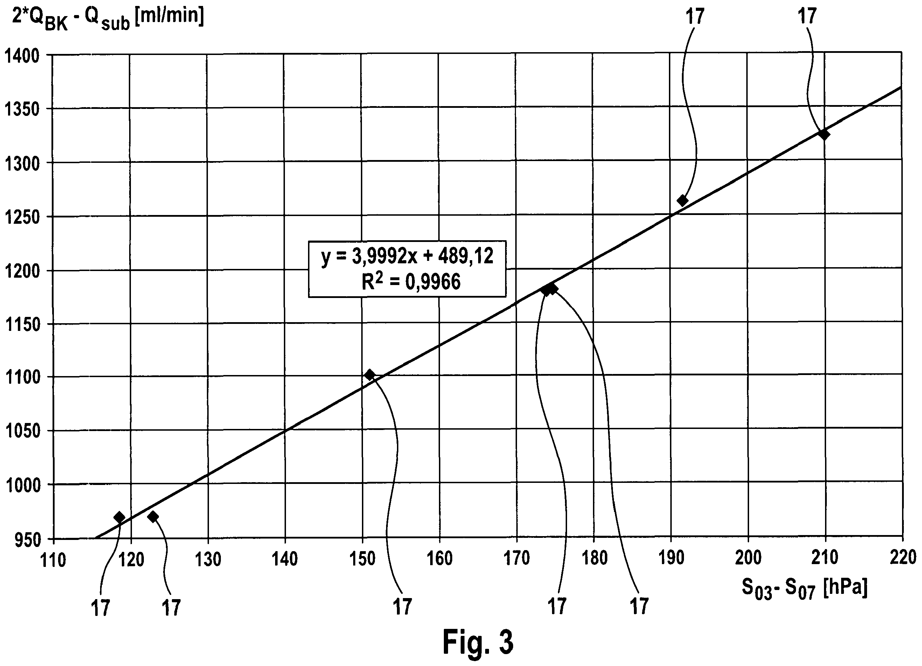

**Fig. 3** zeigt eine Messkurve des zweifachen oder doppelten Dialysatflusses $Q_{BK}$ abzüglich des Substituatflusses $Q_{sub}$ in Abhängigkeit der Druckdifferenz zwischen der ersten Druckmessstelle 9 (Druckmesswert $S_{03}$) und der zweiten Druckmessstelle 13 (Druckmesswert $S_{07}$). Der Dialysatorfluss $Q_d$ beträgt konstant 500 ml/min. Der Blutfluss im Primärkreislauf beträgt $Q_b$ = 450 ml/min.

In Fig. 3 sind sieben Messpunkte 17 aufgetragen, die unter Variation des Substituatflusses $Q_{sub}$ gemessen wurden. Die Messpunkte 17 wurden bei Volumenflüssen $(2*Q_{BK} - Q_{sub})$ zwischen 950 ml/min und 1.350 ml/min und Druckdifferenzwerten $(S_{03} - S_{07})$ zwischen 110 hPa und 220 hPa aufgenommen. Durch die Messpunkte 17 wurde eine Gerade mit der angegebenen Funktion y = f(x) gelegt. Der Korrelationskoeffizient beträgt $R^2$ = 0,9966.

Die Steigung der Geraden stellt den reziproken Strömungswiderstand 1/R dar. Hieraus kann der Substituatfluss $Q_{sub}$ berechnet werden.

Der Substituatfluss $Q_{sub}$ kann weiterhin auch unter Berücksichtigung der nichtlinearen Strömungswiderstände $R_1$, $R_2$ und unter Berücksichtigung der Ultrafiltratrate $Q_{uf}$ (kurz UF-Rate), welche als Volumenstrom im Dialysator 300 aus dem Primärkreislauf 100 in den Sekundärkreislauf 200 übergeht, berechnet werden.

Ersetzt man in Gleichung (4a) die Produkte $Q_d*R_1(Q_d)$ bzw. $Q_{BK}*R_2(Q_{BK})$ durch die Druckdifferenzen, die als Funktion der Flüsse dargestellt werden,

$$F_{1,m}(Q_d) = Q_d * R_1(Q_d) \tag{9a}$$

$$F_{m,2}(Q_{BK} + Q_{UF}) = Q_{BK} * R_2(Q_{BK}) \tag{9b}$$

so erhält man allgemein

$$S_{03} - S_{07} = F_{1,m}(Q_d) + F_{m,2}(Q_{BK} + Q_{UF}) \tag{10}.$$

Mit Hilfe der Umformung

$$S_{03} - S_{07} = F_{1,m}(Q_{BK} + Q_{UF}) + F_{m,2}(Q_{BK} + Q_{UF}) - \left( F_{1,m}(Q_{BK} + Q_{UF}) - F_{1,m}(Q_d) \right)$$

und der Definition

$$F_{1,2}(Q_{BK} + Q_{UF}) = F_{1,m}(Q_{BK} + Q_{UF}) + F_{m,2}(Q_{BK} + Q_{UF})$$

erhält man schließlich

$$S_{03} - S_{07} = F_{1,2}(Q_{BK} + Q_{UF}) - \left( F_{1,m}(Q_{BK} + Q_{UF}) - F_{1,m}(Q_{BK} - Q_{sub}) \right) \tag{11}$$

Die Funktion $F_{1,2}$ liefert den größten Beitrag zur Druckdifferenz $S_{03} - S_{07}$. Bei ausgeschalteter Substitutionspumpe bzw. $Q_{sub} = 0$, und bei UF-Rate $Q_{uf} = 0$ kann $F_{1,2}(Q)$ online bestimmt werden, indem der Bilanzkammerfluss $Q_{BK} = Q$ variiert wird:

$$F_{1,2}(Q) = S_{03} - S_{07} \tag{12}$$

Die Funktion $F_{1,m}(Q)$ kann im Rahmen der Typprüfung für alle Geräte oder durch eine Werkskalibrierung gerätespezifisch bestimmt werden. Alternativ kann die Funktion $F_{1,m}(Q)$ in Abhängigkeit der Funktion $F_{1,2}(Q)$ festgelegt werden, z. B.:

$$F_{1,m}(Q) = 0,5 * F_{1,2}(Q) + k*Q$$

wobei der Term k*Q den Druckabfall am Dialysatfilter 15 berücksichtigt (wegen der Asymmetrie der beiden Strecken), oder, allgemein:

$$F_{1,m}(Q) = a * F_{1,2}(Q) + k*Q$$

In der Regel ist bei der HDF-Behandlung der Dialysatfluss $Q_{BK}$ deutlich größer als der Substituatfluss $Q_{sub}$. Die Differenz $F_{1,m}(Q_{BK} + Q_{UF}) - F_{1,m}(Q_d)$ kann näherungsweise durch das totale Differential $dF_{1,m}$ ersetzt werden. Man erhält dann:

$$S_{03} - S_{07} \approx F_{1,2}\left(Q_{BK} + Q_{UF}\right) - \left(\frac{dF_{1,m}}{dQ}\right)_{(Q_{BK}+Q_{UF}+Q_d)/2} * Q_{sub} \qquad (13)$$

mit:

$F_{1,m}$: Druckverlust als Funktion des Volumenstroms zwischen erster Druckmessstelle 9 und der Mitte des Blutbehandlungsmoduls 300; und

Q: Durchfluss mit dem Wert $(Q_{BK}+Q_{UF}+Q_d)/2$

Da die beiden Funktionen $F_{1,m}(Q)$ und $F_{1,2}(Q)$ bekannt sind, lässt sich die Substituatrate $Q_{sub}$ aus Gleichung (11) oder aus Gleichung (13) berechnen.

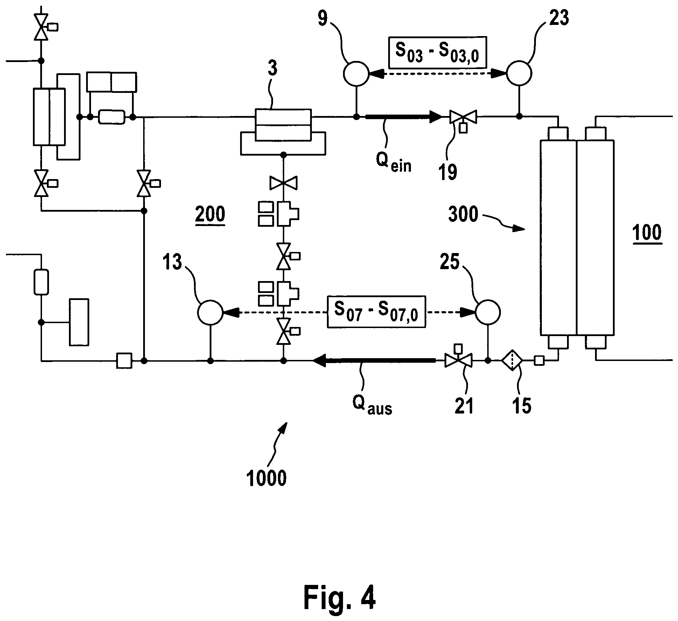

**Fig. 4** basiert auf der Darstellung der Fig. 1 und zeigt eine erfindungsgemäße Blutbehandlungsvorrichtung 1000 einer zweiten Ausführungsform.

Abweichend von der in Fig. 1 dargestellten Blutbehandlungsvorrichtung weist die in Fig. 4 dargestellte Blutbehandlungsvorrichtung 1000 zusätzlich einen dritten Drucksensor 23 als eine dritten Druckmessstelle zum Erheben eines dritten Druckmesswerts $S_{03,0}$ und einen vierten Drucksensor 25 als eine vierte Druckmessstelle zum Erheben eines vierten Druckmesswerts $S_{07,0}$ auf.

Der Substituatfluss $Q_{sub}$ wird auch in diesem zweiten Ausführungsbeispiel - wie in Fig. 1 gezeigt - in den Primärkreislauf eingeführt, obwohl dies aus Gründen der Übersichtlichkeit in Fig. 4 nicht gesondert dargestellt ist.

Durch die zwei zusätzlichen Drucksensoren, d. h. den dritten Drucksensor 23 und den vierten Drucksensor 25, können ein Dialysator-Eingangsfluss $Q_{ein}$ - mittels der Druckdifferenz $S_{03} - S_{03,0}$ - und ein Dialysator-Ausgangsfluss $Q_{aus}$ - mittels der Druckdifferenz $S_{07} - S_{07,0}$ - an definierten Strömungswiderständen ermittelt werden. Im vorliegenden

Beispiel sind die bekannten Strömungswiderstände durch die vorhandenen Ventile 19 und 21 im Sekundärkreislauf 200 realisiert. Erfindungsgemäß vorgesehen ist zum Bestimmen der Strömungswiderstände wie hier beschrieben anstelle von Ventilen alternativ auch der Einsatz dezidierter Drosseln, insbesondere solcher, welche ohne mechanisch bewegte Teile auskommen, und deren Druckabfälle in einem gut messbaren Bereich liegen.

Prinzipiell lässt sich mit dieser Anordnung der Differenzfluss zwischen dem Fluss, der dem Dialysator 300 eingangsseitig zufließt und dem Fluss, der den Dialysator 300 ausgangseitig wieder verlässt, bestimmen.

Neben dem Substituatfluss $Q_{sub}$ (in Fig. 4 nicht dargestellt) werden damit auch die UF-Rate $Q_{UF}$ (siehe die Beschreibung zu Fig. 3) sowie eine Leckrate $Q_{Leck}$ bei einer Fehlbilanz erfasst. Die Summe der Flüsse wird als Transmembranfluss $Q_{tm}$ bezeichnet, der über die Dialysatormembran fließt oder zwischen Primärkreislauf 100 und Sekundärkreislauf 200 im Dialysator ausgetauscht wird.

$$Q_{tm} = Q_{sub} + Q_{UF} + Q_{Leck} \qquad (14)$$

mit:

$Q_{tm}$ - Transmembranfluss
$Q_{sub}$ - Substituatfluss
$Q_{uf}$ - Ultrafiltrationsrate
$Q_{Leck}$ - Leckrate

Ersetzt man den Dialysatfluss $Q_{BK}$ in Gleichung (5) durch die Summe $Q_d + Q_{sub} + Q_{UF} + Q_{Leck}$ so erhält man:

$$S_{03} - S_{07} = Q_d * R_1 + \left(Q_d + Q_{sub} + Q_{UF} + Q_{Leck}\right) * R_2$$

Damit ergibt sich die Leckrate $Q_{Leck}$ zu:

$$Q_{Leck} = \frac{S_{03} - S_{07}}{R_2} - Q_d * \left(1 + \frac{R_1}{R_2}\right) - Q_{sub} - Q_{UF} \qquad (15)$$

Aus der Steigung der Geraden in Fig. 3 ergibt sich der reziproke Widerstand l/R im vorliegenden Beispiel zu:

l/R = 4,0 ml/min / hPa = 320 ml/h/mmHg.

Dieser Wert liegt in der Größenordnung des maximalen Ultrafiltrationskoeffizienten (UFK) von Dialysatoren mit einem $UFK_{max}$ = 200 ml/h/mmHg.

Die Strömungswiderstände $R_1$ und $R_2$ können Konstanten sein. Sie müssen jedoch keine Konstanten sein; sie können vielmehr selbst variieren, beispielsweise in Abhängigkeit vom Durchfluss Q.

Durch einen Druckhaltetest (DHT) kann die Dichtigkeit des Bilanzierkreises getestet werden. Die zuvor registrierte Leckrate $Q_{Leck,0}$ kann als Offset interpretiert werden und nach dem DHT abgezogen werden:

$$Q_{Leck,eff} = Q_{Leck} - Q_{Leck,0} = \frac{\left(S_{03} - S_{07}\right) - \left(S_{03} - S_{07}\right)_0}{R_2} - \left(Q_{sub} - Q_{sub,0}\right) - \left(Q_{UF} - Q_{UF,0}\right) \qquad (16)$$

**Bezugszeichenliste**

| Bezugszeichen | Beschreibung |
|---|---|
| 1000 | Blutbehandlungsvorrichtung |
| 100 | Primärkreislauf |
| 200 | Sekundärkreislauf |
| 300 | Blutbehandlungsmodul, Dialysator |
| $Q_{sub}$ | erster Volumenstrom, Substituatfluss |
| $Q_{BK}$ | zweiter Volumenstrom, Dialysatfluss |
| $Q_d$ | dritter Volumenstrom, Dialysatorfluss |
| $Q_{ein}$ | Dialysator-Eingangsfluss |
| $Q_{aus}$ | Dialysator-Ausgangsfluss |
| $P_{Dm}$ | Druckwert in der Mitte des Blutbehandlungsmoduls 300 |
| 3 | Filter |
| 5 | Pfeil, deutet eine Fluidverbindung an |
| 9 | erster Drucksensor, erste Druckmessstelle, liefert ersten Druckmesswert $S_{03}$ |
| 13 | zweiter Drucksensor, zweite Druckmessstelle, liefert zweiten Druckmesswert $S_{07}$ |

(fortgesetzt)

| Bezugszeichen | Beschreibung |
|---|---|
| 15 | Dialysatfilter |
| 17 | Messpunkte |
| 19 | erstes Ventil |
| 21 | zweites Ventil |
| 23 | dritter Drucksensor, dritte Druckmessstelle, liefert dritten Druckmesswert $S_{03,0}$ |
| 25 | vierter Drucksensor, vierte Druckmessstelle, liefert vierten Druckmesswert $S_{07,0}$ |

**Patentansprüche**

1. Verfahren zum Bestimmen wenigstens eines Volumenstroms in einer Blutbehandlungsvorrichtung (1000), welche aufweist:

   - einen Primärkreislauf (100) zum Leiten des zu behandelnden Bluts;
   - einen Sekundärkreislauf (200) zum Leiten eines Fluids, das zur Blutbehandlung verwendet wird; und
   - ein Blutbehandlungsmodul (300) als Abschnitt des Primärkreislaufs (100) und des Sekundärkreislaufs (200), das dazu vorgesehen ist, Fluide und/oder Stoffe zwischen dem Primärkreislauf (100) und dem Sekundärkreislauf (200) auszutauschen;

   **dadurch gekennzeichnet, dass** die Blutbehandlungsvorrichtung (1000) ferner aufweist:

   - eine als Schlauchleitung oder Verbindungsleitung ausgebildete Fluidverbindung (5) zwischen dem Primärkreislauf (100) und dem Sekundärkreislauf (200), welche zum Einbringen eines ersten Volumenstroms ($Q_{sub}$) aus dem Sekundärkreislauf (200) in den Primärkreislauf (100) vorgesehen ist,

   und **dadurch gekennzeichnet, dass** das Verfahren wenigstens aufweist:

   das Bestimmen des ersten Volumenstroms ($Q_{sub}$) des Sekundärkreislaufs (200) unter Einbeziehung oder Berücksichtigung eines ersten Druckwerts oder Druckmesswerts ($S_{03}$) und eines zweiten Druckwerts oder Druckmesswerts ($S_{07}$) im Sekundärkreislauf (200), wobei der erste Volumenstrom ($Q_{sub}$) ein Teilvolumenstrom eines zweiten Volumenstroms ($Q_{BK}$) ist und das Bestimmen des ersten Volumenstroms ($Q_{sub}$) als Funktion des Strömungswiderstands, des zweiten Volumenstroms ($Q_{BK}$) und der Differenz zwischen zweitem Druckmesswert ($S_{07}$) und erstem Druckmesswert ($S_{03}$) erfolgt, wobei der erste Volumenstrom ($Q_{sub}$) ein Substituatfluss und der zweite Volumenstrom ($Q_{BK}$) ein Dialysatfluss ist.

2. Verfahren zum Bestimmen wenigstens eines Volumenstroms nach Anspruch 1, welches ferner aufweist:

   - Bestimmen eines Volumenstroms ($Q_{ein}$) des Sekundärkreislaufs (200) stromauf des Blutbehandlungsmoduls (300) unter Einbeziehung oder Berücksichtigung einer ersten Druckdifferenz ($S_{03} - S_{03,0}$), und/oder Bestimmen eines Volumenstroms ($Q_{aus}$) des Sekundärkreislaufs (200) stromab des Blutbehandlungsmoduls (300) unter Einbeziehung oder Berücksichtigung einer zweiten Druckdifferenz ($S_{07} - S_{07,0}$) wobei der Volumenstrom stromauf ($Q_{ein}$) und/oder der Volumenstrom stromab ($Q_{aus}$) des Blutbehandlungsmoduls (300) jeweils mittels einer Druckdifferenzbestimmung an definierten Strömungswiderständen ermittelt wird.

3. Verfahren nach Anspruch 1 oder 2, wobei der erste Druckmesswert ($S_{03}$) einen Druck im Sekundärkreislauf (200) an einer ersten Druckmessstelle (9) stromauf des Blutbehandlungsmoduls (300) und stromab einer Abzweigung des ersten Volumenstroms ($Q_{sub}$) aus einem zweiten Volumenstrom ($Q_{BK}$) angibt, und wobei der zweite Druckmesswert ($S_{07}$) einen Druck im Sekundärkreislauf (200) an einer zweiten Druckmessstelle (13) stromab des Blutbehandlungsmoduls (300) angibt.

4. Verfahren nach einem der Ansprüche 1 bis 3, mit dem Schritt:

Bestimmen eines Strömungswiderstands zwischen erster Druckmessstelle (9) und zweiter Druckmessstelle (13) als Funktion des zweiten Volumenstroms ($Q_{BK}$) und der Differenz zwischen zweitem Druckmesswert ($S_{07}$) und erstem Druckmesswert ($S_{03}$).

5. Verfahren nach Anspruch 4, wobei die Funktion zum Bestimmen des Strömungswiderstands im Bereich eines Arbeitspunktes des zweiten Volumenstroms ($Q_{BK}$) und einer Differenz zwischen zweitem Druckmesswert ($S_{07}$) und erstem Druckmesswert ($S_{03}$) linearisiert wird oder wurde.

6. Verfahren nach einem der Ansprüche 1 bis 5, wobei der funktionelle Zusammenhang eines Druckverlusts zwischen erster Druckmessstelle (9) und einem Abschnitt, insbesondere einem mittleren Abschnitt, innerhalb des Blutbehandlungsmoduls (300) in Abhängigkeit des zweiten Volumenstroms ($Q_{BK}$) des Sekundärkreislaufs (200) werksseitig oder vor Beginn der Blutbehandlung bestimmt wird oder wurde.

7. Verfahren nach einem der Ansprüche 1 bis 6, wobei der funktionelle Zusammenhang eines Druckverlusts zwischen erster Druckmessstelle (9) und einem Abschnitt innerhalb des Blutbehandlungsmoduls (300) in Abhängigkeit des zweiten Volumenstroms ($Q_{BK}$) bestimmt wird oder wurde als Funktion des Druckverlusts zwischen erster Druckmessstelle (9) und zweiter Druckmessstelle (13), wiederum in Abhängigkeit des zweiten Volumenstroms ($Q_{BK}$).

8. Verfahren nach einem der vorangegangenen Ansprüche, wobei zum Ermitteln des Druckverlusts zwischen erster Druckmessstelle (9) und zweiter Druckmessstelle (13) berücksichtigt wird oder wurde:

   - Druckverlust zwischen erster Druckmessstelle (9) und zweiter Druckmessstelle (13) in Abhängigkeit des zweiten Volumenstroms ($Q_{BK}$) und des Volumenstroms ($Q_{UF}$), der im Behandlungsmodul (300) vom Primärkreislauf (100) in den Sekundärkreislauf (200) übertritt;
   - totales Differential $dF_{1,m}/dQ$.
   mit $F_{1,m}$ - Druckverlust als Funktion des Volumenstroms zwischen erster Druckmessstelle und der Mitte des Blutbehandlungsmoduls; und
   mit Q Durchfluss mit dem Wert $(Q_{BK}+Q_{UF}+Q_d)/2$, wobei gilt:
   $Q_{BK}$ - zweiter Volumenstrom;
   $Q_{UF}$ - Volumenstrom, der im Blutbehandlungsmodul aus dem Primärkreislauf in den Sekundärkreislauf übertritt; und
   $Q_d$ - zweiter Volumenstrom abzüglich des ersten Volumenstroms.

9. Verfahren nach einem der vorangegangenen Ansprüche, wobei zum Bestimmen eines Lecks oder einer Leckrate resultierend aus unterschiedlich großen Volumenströmen im Sekundärkreislauf (200) stromab und stromauf des Blutbehandlungsmoduls (300) ein funktioneller Zusammenhang der folgenden Parameter angegeben wird:

   - erster Druckmesswert ($S_{03}$);
   - zweiter Druckmesswert ($S_{07}$);
   - Strömungswiderstand zwischen erster Druckmessstelle (9) und einem Abschnitt innerhalb des Blutbehandlungsmoduls (300);
   - Strömungswiderstand zwischen dem Abschnitt innerhalb des Blutbehandlungsmoduls (300) und zweiter Druckmessstelle (13);
   - erster Volumenstrom ($Q_{sub}$);
   - zweiter Volumenstrom ($Q_{BK}$);
   - einem dritten Volumenstrom, der einen Fluidaustausch oder Fluidübertritt über eine Membran zwischen Primärkreislauf (100) und Sekundärkreislauf (200) des Blutbehandlungsmoduls (300) angibt.

10. Verfahren nach einem der vorangegangenen Ansprüche, wobei ein Offset für Volumenstrombestimmungen im Sekundärkreislauf (200) mittels der folgenden Schritte ermittelt wird:

   - Überprüfen ob Dichtigkeit des Sekundärkreislaufs (200) gegeben ist;
   - Überprüfen, ob im Blutbehandlungsmodul (300) kein Fluid zwischen Primärkreislauf (100) und Sekundärkreislauf (200) ausgetauscht wird;
   - Ermitteln einer Differenz zwischen dem Volumenstrom stromauf ($Q_{ein}$) und dem Volumenstrom stromab ($Q_{aus}$) des Blutbehandlungsmoduls (300) im Sekundärkreislauf (200); und
   - Festlegen der ermittelten Volumenstromdifferenz als Offset.

**11.** Verfahren nach Anspruch 10, wobei das Offset bei der Bestimmung weiterer Volumenströme berücksichtigt.

**12.** Verfahren nach einem der vorangegangenen Ansprüche, wobei die Blutbehandlungsvorrichtung (1000) zur Hämodialyse oder zur Hämodiafiltration eingerichtet ist.

**13.** Blutbehandlungsvorrichtung (1000) aufweisend oder verbunden mit

- einem Primärkreislauf (100) zum Leiten des zu behandelnden Bluts;
- einem Sekundärkreislauf (200) zum Leiten eines Fluids, das zur Blutbehandlung verwendet wird;
- einem Blutbehandlungsmodul (300) als Abschnitt des Primärkreislaufs (100) und des Sekundärkreislaufs (200), vorgesehen für einen Austausch von Fluiden und/oder Stoffen zwischen dem Primärkreislauf (100) und dem Sekundärkreislauf (200);

**dadurch gekennzeichnet, dass** die Blutbehandlungsvorrichtung (1000) ferner aufweist:

- eine als Schlauchleitung oder Verbindungsleitung ausgebildete Fluidverbindung (5) zwischen dem Primärkreislauf (100) und dem Sekundärkreislauf (200), welche zum Einbringen des ersten Volumenstroms ($Q_{sub}$) aus dem Sekundärkreislauf (200) in den Primärkreislauf (100) vorgesehen ist; und
- wenigstens einer Recheneinrichtung , welche geeignet und konfiguriert ist zum Durchführen des Verfahrens nach wenigstens einem der Ansprüche 1 bis 12.

**14.** Blutbehandlungsvorrichtung (1000) nach Anspruch 13, ferner aufweisend:

- eine Einrichtung zum Bestimmen des ersten Volumenstroms ($Q_{sub}$) des Sekundärkreislaufs (200) unter Einbeziehung oder Berücksichtigung eines ersten Druckwerts oder Druckmesswerts ($S_{03}$) und eines zweiten Druckwerts oder Druckmesswerts ($S_{07}$) im Sekundärkreislauf (200), wobei der erste Volumenstrom ($Q_{sub}$) ein Teilvolumenstrom eines zweiten Volumenstroms ($Q_{BK}$) ist und das Bestimmen des ersten Volumenstroms ($Q_{sub}$) als Funktion des Strömungswiderstands, des zweiten Volumenstroms ($Q_{BK}$) und der Differenz zwischen zweitem Druckmesswert ($S_{07}$) und erstem Druckmesswert ($S_{03}$)erfolgt, wobei der erste Volumenstrom ($Q_{sub}$) ein Substituatfluss und der zweite Volumenstrom ($Q_{BK}$) ein Dialysatfluss ist.;

und

- eine Einrichtung zum Bestimmen eines Volumenstroms ($Q_{ein}$) des Sekundärkreislaufs (200) stromauf des Blutbehandlungsmoduls (300) unter Einbeziehung oder Berücksichtigung einer ersten Druckdifferenz ($S_{03} - S_{03,0}$), und/oder Bestimmen eines Volumenstroms ($Q_{aus}$) des Sekundärkreislaufs (200) stromab des Blutbehandlungsmoduls (300) unter Einbeziehung oder Berücksichtigung einer zweiten Druckdifferenz ($S_{07} - S_{07,0}$) wobei der Volumenstrom stromauf ($Q_{ein}$) und/oder der Volumenstrom stromab ($Q_{aus}$) des Blutbehandlungsmoduls (300) jeweils mittels einer Druckdifferenzbestimmung an definierten Strömungswiderständen ermittelt wird.

**15.** Blutbehandlungsvorrichtung (1000) nach einem der vorangegangenen Ansprüche 13 bis 14 mit:

- einem ersten, schließbaren Ventil (19) stromauf des Blutbehandlungsmoduls (300);
- einem zweiten, schließbaren Ventil (21) stromab des Blutbehandlungsmoduls (300);
- optional eine Einrichtung geeignet und/oder konfiguriert zum Prüfen, ob der erste Volumenstrom (Qsub) Null ist;
- eine Einrichtung geeignet und/oder konfiguriert, um das Fluidsystem stromauf des ersten Ventils (19) und/oder stromab des zweiten Ventils (21) mit einem Überdruck zu beaufschlagen;
- eine Einrichtung geeignet und/oder konfiguriert zum Überprüfen, ob sich der Überdruck im Fluidsystem innerhalb einer vorgegebenen Zeitspanne verändert und/oder ob ein Druckabfall innerhalb eines vorgegebenen Bereichs liegt; und
- eine Einrichtung geeignet und/oder konfiguriert zum Feststellen der Dichtigkeit des Sekundärkreislaufs (200) für den Fall, dass der Überdruck innerhalb der vorgegebenen Zeitspanne beibehalten bleibt und/oder dass der Druckabfall innerhalb des vorgegebenen Bereichs liegt.

**Claims**

**1.** A method for determining at least one volume flow in a blood treatment apparatus (1000), which comprises:

- a primary circuit (100) for conducting the blood to be treated;
- a secondary circuit (200) for conducting a fluid that is used for the blood treatment; and
- a blood treatment module (300) as section of the primary circuit (100) and the secondary circuit (200), which is provided for exchanging fluids and/or substances between the primary circuit (100) and the secondary circuit (200);

**characterized in that** the blood treatment apparatus (1000)comprises further:

- a fluid connection (5) between the primary circuit (100) and the secondary circuit (200) which is embodied as a tube line or connecting line and provided for introducing a first volume flow ($Q_{sub}$) from the secondary circuit (200) into the primary circuit (100);

and **characterized in that** the method at least comprises:

determining the first volume flow ($Q_{sub}$) of the secondary circuit (200) taking into account or considering a first pressure value or pressure measurement ($S_{03}$) and a second pressure value or pressure measurement ($S_{07}$) in the secondary circuit (200), wherein the first volume flow ($Q_{sub}$) is a partial volume flow of a second volume flow ($Q_{BK}$) and the determining of the first volume flow ($Q_{sub}$) occurs as function of the flow resistance, the second volume flow ($Q_{BK}$) and the difference between the second pressure measurement ($S_{07}$) and the first pressure measurement ($S_{03}$), wherein the first volume flow ($Q_{sub}$) is a substitute flow and the second volume flow ($Q_{BK}$) is a dialysate flow.

2. The method for determining at least one volume flow according to claim 1, comprising further:

- determining a volume flow ($Q_{ein}$) of the secondary circuit (200) upstream from the blood treatment module (300) taking into account or considering a first pressure difference ($S_{03}$ - $S_{03,0}$), and/or determining a volume flow ($Q_{aus}$) of the secondary circuit (200) downstream from the blood treatment module (300) taking into account or considering a second pressure difference ($S_{07}$ - $S_{07,0}$), wherein the volume flow upstream ($Q_{ein}$) and/or the volume flow downstream ($Q_{aus}$) from the blood treatment module (300) is each detected by means of a pressure difference determination at defined flow resistances.

3. The method according to claim 1 or 2, wherein the first pressure measurement ($S_{03}$) specifies a pressure in the secondary circuit (200) at a first pressure measurement location (9) upstream from the blood treatment module (300) and downstream from a branch-off of the first volume flow ($Q_{sub}$) from a second volume flow ($Q_{BK}$) and wherein the second pressure measurement ($S_{07}$) specifies a pressure in the secondary circuit (200) at a second pressure measurement location (13) downstream from the blood treatment module (300).

4. The method according to anyone of the claims 1 to 3, with the step:

determining a flow resistance between first pressure measurement location (9) and second pressure measurement location (13) as function of the second volume flow ($Q_{BK}$) and the difference between second pressure measurement ($S_{07}$) and first pressure measurement ($S_{03}$).

5. The method according to claim 4, wherein the function for determining the flow resistance is or was linearized in the area of an operating point of the second volume flow ($Q_{BK}$) and a difference between second pressure measurement ($S_{07}$) and first pressure measurement ($S_{03}$).

6. The method according to any one of the claims 1 to 5, wherein the functional connection of a pressure loss between first pressure measurement location (9) and a section, in particular a central section, within the blood treatment module (300) is or was determined by the factory or before the beginning of the blood treatment depending on the second volume flow ($Q_{BK}$) of the secondary circuit (200).

7. The method according to any one of the claims 1 to 6, wherein the functional connection of a pressure loss between first pressure measurement location (9) and a section within the blood treatment module (300) is determined depending on the second volume flow ($Q_{BK}$) or was determined as function of the pressure loss between first pressure measurement location (9) and second pressure measurement location (13), again depending on the second volume flow ($Q_{BK}$).

8. The method according to any one of the preceding claims, wherein for determining the pressure loss between first pressure measurement location (9) and second pressure measurement location (13) the following is or was considered:

- pressure loss between first pressure measurement location (9) and second pressure measurement location (13) depending on the second volume flow ($Q_{BK}$) and the volume flow ($Q_{UF}$) that transfers from the primary circuit (100) into the secondary circuit (200) in the treatment module (300);
- total differential $dF_{1,m}/dQ$.

with $F_{1,m}$ being the pressure drop as function of the volume flow between the first pressure measurement location and the middle of the blood treatment module; and with Q being the flow rate with the value $(Q_{BK}+Q_{UF}+Q_d)/2$, wherein:

$Q_{BK}$ is the second volume flow;
$Q_{UF}$ is the volume flow that transfers from the primary circuit in the secondary circuit; and
$Q_d$ is the second volume flow minus the first volume flow.

9. The method according to any one of the preceding claims, wherein for identifying a leakage or a leakage rate resulting from the differently sized volume flows in the secondary circuit (200) downstream and upstream from the blood treatment module (300) a functional connection of the following parameters is specified:

- first pressure measurement ($S_{03}$);
- second pressure measurement ($S_{07}$)
- flow resistance between first pressure measurement location (9) and a section within the blood treatment module (300);
- flow resistance between the section within the blood treatment module (300) and second pressure measurement location (13);
- first volume flow ($Q_{sub}$);
- second volume flow ($Q_{BK}$);
- a third volume flow which specifies a fluid exchange or fluid transfer via a membrane between primary circuit (100) and secondary circuit (200) of the blood treatment module (300).

10. The method according to any one of the preceding claims, wherein an offset for volume flow determinations in the secondary circuit (200) is detected by means of the following steps:

- checking whether tightness of the secondary circuit (200) is given;
- checking whether no fluid is exchanged between primary circuit (100) and secondary circuit (200) in the blood treatment module (300);
- determining a difference between the volume flow upstream ($Q_{ein}$) and the volume flow downstream ($Q_{aus}$) from the blood treatment module (300) in the secondary circuit (200); and
- setting the detected volume flow difference as offset.

11. The method according to claim 10, wherein the offset is taken into account in the determination of further volume flows.

12. The method according to any one of the preceding claims, wherein the blood treatment apparatus (1000) is set up for hemodialysis or hemodiafiltration.

13. A blood treatment apparatus (1000) comprising or connected with

- a primary circuit (100) for conducting the blood to be treated;
- a secondary circuit (200) for conducting a fluid that is used for the blood treatment;
- a blood treatment module (300) as section of the primary circuit (100) and the secondary circuit (200), provided for an exchange of fluids and/or substances between the primary circuit (100) and the secondary circuit (200);

**characterized in that** the blood treatment apparatus (1000) further comprises:

- a fluid connection (5) between the primary circuit (100) and the secondary circuit (200), which is embodied as a tube line or connection line and provided for introducing a first volume flow (Qsub) from the secondary circuit (200) into the primary circuit (100); and

- at least one calculating device suitable and configured for performing the method according to at least one of the claims 1 to 12.

**14.** The blood treatment apparatus (1000) according to claim 13 further comprising:

- a device for determining the first volume flow (Qsub) of the secondary circuit (200) taking into account or considering a first pressure value or pressure measurement (S03) and a second pressure value or pressure measurement (S07) in the secondary circuit (200), wherein the first volume flow (Qsub) is a partial volume flow of a second volume flow (QBK) and the determining of the first volume flow (Qsub) occurs as function of the flow resistance, the second volume flow (QBK) and the difference between the second pressure measurement (S07) and the first pressure measurement (S03), wherein the first volume flow (Qsub) is a substituate flow and the second volume flow (QBK) is a dialysate flow; and
- a device for determining a volume flow (Qein) of the secondary circuit (200) upstream from the blood treatment module (300) taking into account or considering a first pressure difference (S03 - S03,0), and/or for determining a volume flow (Qaus) of the secondary circuit (200) downstream from the blood treatment module (300) taking into account or considering a second pressure difference (S07 - S07,0), wherein the volume flow upstream (Qein) and/or the volume flow downstream (Qaus) from the blood treatment module (300) is each detected by means of a pressure difference determination at defined flow resistances.

**15.** The blood treatment apparatus (1000) according to claim 13 or 14 with:

- a first closable valve (19) upstream from the blood treatment module (300);
- a second closable valve (21) downstream from the blood treatment module (300);
- optionally a device provided and/or configured for checking if the first volume flow (Qsub) is zero;
- a device provided and/or configured for pressurizing the fluid system upstream from the first valve (19) and/or downstream from the second valve (21) with an overload pressure;
- a device provided and/or configured for checking if the overload pressure in the fluid system changes within a predetermined time range and/or if a pressure drop lies within a predetermined range; and
- a device provided and/or configured for determining the tightness of the second blood circuit (200) for the case that the overload pressure stays within the predetermined time range and/or the pressure drop lies within the predetermined range.

## Revendications

**1.** Un procédé pour déterminer au moins un débit volumique dans un appareil de traitement du sang (1000), comprenant :

- un circuit primaire (100) destiné à diriger le sang devant être traité ;
- un circuit secondaire (200) destiné à diriger un fluide qui est utilisé pour le traitement du sang ;
- un module de traitement du sang (300) formant une section du circuit primaire (100) et du circuit secondaire (200) et étant destiné à échanger des fluides et / ou des substances entre le circuit primaire (100) et le circuit secondaire (200) ;

**caractérisé en ce que** l'appareil de traitement du sang (1000) comprend de plus :

- une communication fluidique (5) entre le circuit primaire (100) et le circuit secondaire (200) établie par une conduite flexible ou une conduite de liaison et destinée à introduire un premier débit volumique (QSub) du circuit secondaire (200) dans le circuit primaire (100) ;

et **caractérisé en ce que** le procédé comprend au moins :

- la détermination du premier débit volumique (QSub) du circuit secondaire (200) en considérant ou en tenant compte d'une première valeur ou mesure de pression (S03) et d'une seconde valeur ou mesure de pression (S07) dans le circuit secondaire (200), où le premier débit volumique (QSub) est un débit volumique partiel d'un deuxième débit volumique (QBK) et où la détermination du premier débit volumique (QSub) se fait en fonction de la résistance à l'écoulement, du second débit volumique(QBK) et de la différence entre la seconde mesure de pression (S07) et la première mesure de pression (S03), où le premier débit volumique (QSub) est un flux de substitut et le second débit volumique (QBK) est un flux de dialysat.

2. Le procédé pour déterminer au moins un débit volumique selon la première revendication, comprenant de plus :

- la détermination d'un débit volumique (Qein) du circuit secondaire (200) en amont du module de traitement du sang (300) en considérant ou en tenant compte d'une première différence de pression (S03-S03,0), et / ou la détermination d'un débit volumique (Qaus) du circuit secondaire (200) en aval du module de traitement du sang (300) en considérant ou en tenant compte d'une seconde différence de pression (S07-S07,0), où le débit volumique en amont (Qein) et / ou le débit volumique en aval (Qaus) du module de traitement du sang (300) est déterminé respectivement au moyen de la détermination d'une différence de pression pour des résistances à l'écoulement définies.

3. Le procédé selon la première ou la seconde revendication, dans lequel la première mesure de pression (S03) indique une pression dans le circuit secondaire (200) à un premier point de mesure de pression (9) en amont du module de traitement du sang (300) et en aval d'une branche du premier débit volumique (QSub) partant du second débit volumique (QBK), et dans lequel la seconde mesure de pression (S07) indique une pression dans le circuit secondaire (200) à un second point de mesure de pression (13) en aval du module de traitement du sang (300).

4. Le procédé selon l'une des revendications 1 à 3, comprenant l'étape consistant à :

- déterminer une résistance à l'écoulement entre le premier point de mesure de pression (9) et le second point de mesure de pression (13) en fonction du second débit volumique (QBK) et de la différence entre la seconde mesure de pression (S07) et la première mesure de pression (S03).

5. Le procédé selon la revendication 4, dans lequel la fonction pour déterminer la résistance à l'écoulement aux alentours d'un point de fonctionnement du second débit volumique (QBK) et d'une différence entre la seconde mesure de pression (S07) et la première mesure de pression (S03) est ou a été linéarisée.

6. Le procédé selon l'une quelconque des revendications 1 à 5, dans lequel la corrélation fonctionnelle d'une perte de pression entre le premier point de mesure de pression (9) et une section interne du module de traitement du sang (300), en particulier une section centrale, est ou a été déterminée en usine ou avant le début du traitement du sang en fonction du second débit volumique (QBK) du circuit secondaire (200).

7. Le procédé selon l'une quelconque des revendications 1 à 6, dans lequel la corrélation fonctionnelle d'une perte de pression entre le premier point de mesure de pression (9) et une section interne du module de traitement du sang (300) est déterminée en fonction du second débit volumique (QBK) ou a été déterminée en fonction de la perte de pression entre le premier point de mesure de pression (9) et le second point de mesure de pression (13), ceci à son tour en fonction du second débit volumique (QBK).

8. Le procédé selon l'une quelconque des revendications précédentes, dans lequel, afin de déterminer la perte de pression entre le premier point de mesure de pression (9) et le second point de mesure de pression (13), il est ou a été tenu compte :

- de la perte de pression entre le premier point de mesure de pression (9) et le second point de mesure de pression (13) en fonction du second débit volumique (QBK) et du débit volumique (QUF) qui est transféré dans le module de traitement du sang (300) du circuit primaire (100) vers le circuit secondaire (200) ;
- de la différentielle totale $dF1,m/dQ$.

F1,m étant la perte de pression en fonction du débit volumique entre le premier point de mesure de pression et le centre du module de traitement du sang ; et
Q étant le débit ayant pour valeur (QBK+QUF+ Qd)/2, où :

QBK est le second débit ;
QUF est le débit volumique étant transféré dans le module de traitement du sang du circuit primaire vers le circuit secondaire ; et
Qd est le second débit volumique après déduction du premier débit volumique.

9. Le procédé selon l'une quelconque des revendications précédentes, dans lequel, afin de déterminer une fuite ou un taux de fuite résultant de débits différents dans le circuit secondaire (200) en aval et en amont du module de traitement du sang (300), une corrélation fonctionnelle des paramètres suivants est indiquée :

- la première mesure de pression (S03) ;
- la seconde mesure de pression (S07) ;
- la résistance à l'écoulement entre le premier point de mesure de pression (9) et une section interne du module de traitement du sang (300) ;
- la résistance à l'écoulement entre la section interne du module de traitement du sang (300) et le second point de mesure de pression (13) ;
- le premier débit volumique (QSub)
- le second débit volumique (QBK) ;
- un troisième débit volumique indiquant un échange de fluides ou un transfert de fluides au travers d'une membrane entre le circuit primaire (100) et le circuit secondaire (200) du module de traitement du sang (300).

10. Le procédé selon l'une quelconque des revendications précédentes, dans lequel un offset pour les déterminations de fluide volumique dans le circuit secondaire (200) est obtenu au moyen des étapes suivantes consistant à :

- vérifier l'étanchéité du circuit secondaire (200) ;
- vérifier qu'aucun fluide ne soit échangé entre le circuit primaire (100) et le circuit secondaire (200) dans le module de traitement du sang (300) ;
- déterminer une différence entre le débit volumique en amont (Qein) et le débit volumique en aval (Qaus) du module de traitement du sang (300) dans le circuit secondaire (200) ; et
- définir la différence de fluide volumique déterminée en tant qu'offset.

11. Le procédé selon la revendication 10, dans lequel l'offset est pris en compte lors de la détermination d'autres débits volumiques.

12. Le procédé selon l'une quelconque des revendications précédentes, dans lequel l'appareil de traitement du sang (1000) est équipé pour l'hémodialyse ou l'hémodiafiltration.

13. Un appareil de traitement du sang (1000) comprenant ou étant connecté à :

- un circuit primaire (100) destiné à diriger le sang devant être traité ;
- un circuit secondaire (200) destiné à diriger un fluide utilisé pour le traitement du sang ;
- un module de traitement du sang (300) formant une section du circuit primaire (100) et du circuit secondaire (200) et étant destiné à l'échange de fluides et / ou de substances entre le circuit primaire (100) et le circuit secondaire (200) ;

**caractérisé en ce que** l'appareil de traitement du sang (1000) comprend de plus :

- une communication fluidique (5) entre le circuit primaire (100) et le circuit secondaire (200) établie par une conduite flexible ou une conduite de liaison, destinée à introduire un premier débit volumique (QSub) du circuit secondaire (200) dans le circuit primaire (100) ; et
- au moins un dispositif de calcul destiné et configuré pour exécuter le procédé selon au moins l'une des revendications 1 à 12.

14. L'appareil de traitement du sang (1000) selon la revendication 13, comprenant de plus :

- un dispositif destiné à déterminer le premier débit volumique (QSub) du circuit secondaire (200) en considérant ou en tenant compte d'une première valeur ou mesure de pression (S03) et d'une seconde valeur ou mesure de pression (S07) dans le circuit secondaire (200), où le premier débit volumique (QSub) est un débit volumique partiel d'un deuxième débit volumique (QBK), où la détermination du premier débit volumique (QSub) s'effectue en fonction de la résistance à l'écoulement, du second débit volumique (QBK) et de la différence entre la seconde mesure de pression (S07) et la première mesure de pression (S03), et où le premier débit volumique (QSub) est un flux de substitut et le second débit volumique (QBK) est un flux de dialysat ;

et

- un dispositif destiné à déterminer un débit volumique (Qein) du circuit secondaire (200) en amont du module de traitement du sang (300) en considérant ou en tenant compte d'une première différence de pression (S03-S03, 0) et / ou destiné à déterminer un débit volumique (Qaus) du circuit secondaire (200) en aval du module de traitement du sang (300) en considérant ou en tenant compte d'une seconde différence de pression (S07-S07, 0), où le débit volumique en amont (Qein) et / ou le débit volumique en aval (Qaus) du module de traitement du sang (300) est déterminé respectivement au moyen de la détermination d'une différence de pression pour des résistances à l'écoulement définies.

**15.** L'appareil de traitement du sang (1000) selon l'une quelconque des revendications 13 à 14 avec :

- un premier clapet fermable (19) en amont du module de traitement du sang (300) ;
- un second clapet fermable (21) en aval du module de traitement du sang (300) ;
- éventuellement un dispositif destiné à et / ou configuré pour vérifier si le premier débit fluidique (QSub) est égal à zéro ;
- un dispositif destiné à et / ou configuré pour appliquer une surpression en amont du premier clapet (19) et / ou en aval du second clapet (21) ;
- un dispositif destiné à et / ou configuré pour vérifier si la surpression dans le système fluidique change pendant un intervalle de temps prédéterminé et / ou pour vérifier si une perte de pression se situe dans un domaine prédéterminé ; et
- un dispositif destiné à et / ou configuré pour constater l'étanchéité du circuit secondaire (200) pour le cas où la surpression reste maintenue pendant l'intervalle de temps prédéterminé et / ou pour le cas où la perte de pression se situe dans le domaine prédéterminé.

Fig. 1

Fig. 2

Fig. 3

$$y = 3{,}9992x + 489{,}12$$
$$R^2 = 0{,}9966$$

x-axis: $S_{03} - S_{07}$ [hPa]

y-axis: $2 * Q_{BK} - Q_{sub}$ [ml/min]

24

**9** **23**

$S_{03} - S_{03,0}$

**3**

$Q_{ein}$ **19**

**200** **300** **100**

**13** **25**

$S_{07} - S_{07,0}$

$Q_{aus}$ **21** **15**

**1000**

# Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008125894 A1
- US 5660722 A
- US 2003136181 A1